# EUROPEAN PATENT APPLICATION

(11) **EP 3 933 046 A1**
(43) Date of publication of application: **05.01.2022**
(21) Application number: 20382599.7
(22) Date of filing: 03.07.2020
(51) Int. Cl.: C12Q 1/68, C12Q 1/689

(54) **METHOD FOR COLORIMETRIC DETECTION OF BACTERIA IN FOOD SAMPLES**

(71) Applicant: Consejo Superior de Investigaciones Científicas (CSIC), 28006 Madrid (ES); Donostia International Physics Center, 20018 Donostia - San Sebastián (Gipuzkoa) (ES); Centro Nacional de Technología y Seguridad alimentaria (CNTA), 31570 San Adrián (ES); Universidad del País Vasco - Euskal Herriko Unibertsitatea, 48160 Leioa (Bizkaia) (ES)
(72) Inventor: GRILLÓ DOLSET, María Jesús, 31192 Mutilva (Navarra) (ES); GARRIDO GONZÁLEZ, Victoria Eugenia, 31192 Mutilva (Navarra) (ES); AIZPURUA IRIAZABAL, Francisco Javier, 20018 Donostia - San Sebastián (Gipuzkoa) (ES); SANROMÁN IGLESIAS, María, 20018 Donostia-San Sebastián (GIPUZKOA) (ES); GRZELCZAK, Marek, 20018 Donostia-San Sebastián (GIPUZKOA) (ES); ECHEVERRÍA GOÑI, Inés, 31570 San Adrián (NAVARRA) (ES)
(74) Representative: Pons

(57) **Abstract**

Method for colorimetric detection of bacterial nucleases in biological and food samples through the use of a hemi-methylated duplex linker and functionalized nanoparticles which, depending if nucleases are present in the sample or not, disperse or aggregate leading to a naked eye visible change in the colour of the solution.

SEQUENCE LISTING

<110> Consejo Superior Investigaciones Científicas
Universidad del País Vasco - Euskal Herriko Unibertsitatea
Donostia International Physics Center
Centro Nacional de Tecnología y Seguridad Alimentaria

<120> Method for colorimetric detection of bacteria in food samples

<130> 1641.1477

<160> 14

<170> PatentIn version 3.5

<210> 1
<211> 18
<212> DNA
<213> Artificial Sequence

<220>
<223> Oligonucleotide probe A

<220>
<221> misc_feature
<222> (1)..(1)
<223> 3'-Thiol-Spacer18

<400> 1
aacgactcat attaacaa 18

<210> 2
<211> 18
<212> DNA
<213> Artificial Sequence

<220>
<223> Oligonucleotide Probe B

<220>
<221> misc_feature
<222> (1)..(1)
<223> 3'-Thiol-Spacer18

<400> 2
tagtctcatt tatgctat 18

<210> 3
<211> 30
<212> DNA
<213> Artificial Sequence

<220>
<223> Oligonucleotide Linker A DNA

<400> 3
gtaagtagta gattgttaat atgagtcgtt 30

<210>  4
<211>  30
<212>  RNA
<213>  Artificial Sequence

<220>
<223>  Oligonucleotide Linker A RNA

<400>  4
guaaguagua gauuguuaau augagucguu                                        30


<210>  5
<211>  30
<212>  RNA
<213>  Artificial Sequence

<220>
<223>  Oligonucleotide Linker A 2'OMe


<220>
<221>  misc_feature
<222>  (1)..(30)
<223>  2'-O-Methylation

<400>  5
guaaguagua gauuguuaau augagucguu                                        30


<210>  6
<211>  30
<212>  DNA
<213>  Artificial Sequence

<220>
<223>  Oligonucleotide Linker B DNA

<400>  6
tctactactt acatagcata aatgagacta                                        30


<210>  7
<211>  30
<212>  RNA
<213>  Artificial Sequence

<220>
<223>  Oligonucleotide Linker B RNA

<400>  7
ucuacuacuu acauagcaua aaugagacua                                        30


<210>  8
<211>  30
<212>  RNA
<213>  Artificial Sequence

<220>
<223>  Oligonucleotide Linker B 2'OMe

<220>
<221>  misc_feature
<222>  (1)..(30)
<223>  2'-O-Methylation

<400>  8
ucuacuacuu acauagcaua aaugagacua                                        30


<210>  9
<211>  30
<212>  DNA
<213>  Artificial Sequence

<220>
<223>  Oligonucleotide Linker C DNA

<400>  9
gcaagcagca gattgttaat atgagtcgtt                                        30


<210>  10
<211>  30
<212>  RNA
<213>  Artificial Sequence

<220>
<223>  Oligonucleotide Linker C RNA

<400>  10
gcaagcagca gauuguuaau augagucguu                                        30


<210>  11
<211>  30
<212>  RNA
<213>  Artificial Sequence

<220>
<223>  Oligonucleotide Linker C 2'OMe


<220>
<221>  misc_feature
<222>  (1)..(30)
<223>  2'-O-Methylation

<400>  11
ucugcugcuu gcauagcaua aaugagacua                                        30


<210>  12
<211>  30
<212>  DNA
<213>  Artificial Sequence

<220>
<223>  Oligonucleotide Linker E DNA

<400>  12
gaaagcagga gattgttaat atgagtcgtt                                        30

<210>  13
<211>  30
<212>  RNA
<213>  Artificial Sequence

<220>
<223>  Oligonucleotide Linker E DNA

<400>  13
gaaagcagga gauuguuaau augagucguu                                        30


<210>  14
<211>  30
<212>  RNA
<213>  Artificial Sequence

<220>
<223>  Oligonucleotide Linker F 2'OMe


<220>
<221>  misc_feature
<222>  (1)..(30)
<223>  2'-O-Methylation

<400>  14
ucuccugcuu ucauagcaua aaugagacua                                        30

## Description

The invention relates to a method for colorimetric detection of bacterial nucleases in food samples through the use of a hemi-methylated duplex linker and functionalized nanoparticles in solution which disperse or aggregate, in the presence or absence of bacterial nucleases, respectively, leading to a visible change in the colour of the solution.

### BACKGROUND ART

Infectious foodborne diseases give rise to real concerns on the public health, especially considering the steadily increasing speed at which food moves through the production and distribution chain. It is then of paramount importance to develop a fast, cost-efficient and easy-to-operate diagnostic method able to detect the main foodborne pathogens with adequate sensitivity and specificity. Among foodborne pathogens, *Salmonella spp.* and *Staphylococcus aureus* (*S. aureus*), require special attention. In particular, only the former is responsible for 100,000 deaths every year in Europe. The accepted methods for detection of these bacteria involve well-standardized microbiological culture techniques that, however, have some inconveniences such as high price, long-time to obtain the results, low sensitivity, cost-labour- and time-consuming, repeated manipulation of infectious samples and the need of trained personnel. As a matter of fact, *Salmonella* detection is regulated by ISO-based standard (ISO 6579:2002) that consists of six experimental steps (pre-enrichment, selective enrichment, plating-out, purification, confirmation and serotyping), requiring nearly 6 days to determine the presence of bacteria. Such a long and complex protocol hampers early detection of *Salmonella,* making urgent the need for rapid sensors to ensure detection of bacteria without specialized training and equipment.

Recently, several fluorescent assays have been proposed for specific targeting of microorganism using chemically modified nucleic acids that undergo selective fragmentation in the presence of secreted nuclease as biomarker. The document WO2013033436A1 describes the rapid detection of microbial-associated nuclease activity with chemically modified nuclease substrates. This document describes an oligonucleotide probe for detecting a microbial endonuclease comprising at one end a fluorescence reporter and at the other end a fluorescence quencher. The probe contains at least a nuclease cleavable site that when cleaved, fluorescence is emitted due to the reporter being released from the quencher. This method, therefore, uses the presence of nucleases in the sample as a biomarker of the presence of bacteria in the sample.

In a similar approach Isabel Machado *et al.* (Isabel Machado, et al. Analytica Chimica Acta. 2019, 1054, 157 - 166) reports the use of nucleic probes for the detection of live *Salmonella* through nuclease activity, again using nuclease activity as a biomarker. Such probes are, as above, modified with a fluorescence molecule at one end and with a fluorescence quencher at the other end. The degradation of the probe due to the nucleases leads to the release of the fluorescence probe away from the quencher and therefore emission of fluorescence.

Other methods have been developed to avoid the need to read fluorescence. Such methods use gold nanoparticles which exhibit unique optical properties, arising from localised surface plasmon resonance (LSPR) in the visible spectral range, rendering them a convenient colorimetric signal transducer in the detection of pathogens. Accordingly, the U.S. patent document US20170122938 discloses a method for detecting the micrococcal bacteria nuclease of *Staphylococcus* using gold nanoparticles functionalized with a single-stranded nucleic acid. The presence of the nucleic acid at the surface of the gold nanoparticles stops these from aggregating in solution. In the presence of the micrococcal nuclease, such nucleic acid strands are cleaved by the nuclease action, and the nanoparticles aggregate leading to a change in the colour of the solution.

Another type of nanoparticles that have proved useful for the detection of bacterial nucleases are nanoparticles of metal oxide, which possess magnetic properties. The U.S. patent document US200309209A1 describes the use of nanoparticles of metal oxide functionalized with an aggregation moiety, like a nucleic acid which acts as a target for the nucleases, whose level of aggregation can be detected by a change in the spin-spin relaxation time, and the absence of aggregates indicates the presence of nucleases on the sample.

Despite allowing the detection of bacterial presence in samples through the detection of bacterial nucleases as biomarkers, the mentioned methods suffer from insufficiencies in their applicability and sensitivity. On one hand the methods based on fluorescence or magnetic spin readouts require specialized equipment to readout the results of the assay. On the other hand, the colorimetric method based on gold nanoparticles suffers from lack of sensitivity and is prone to erroneous readouts, since the aggregation of the nanoparticles is dependent on the state of the single stranded nucleic acid and its accessibility by the nuclease, which can be altered due, for example, to secondary structures of the DNA due to its high content of GC bases or due to suboptimal incubating conditions.

### DESCRIPTION OF THE INVENTION

In order to overcome the limitations of other methods, the present invention discloses a novel method for detection of bacterial presence in food samples by using bacterial nucleases as biomarkers. The method of the present invention makes use of functionalized nanoparticles to allow for a colorimetric readout, avoiding the need of specialized equipment to readout the assay. Therefore, the method is based on the aggregation of nanoparticles, being that such aggregation is control by the oligonucleotide probes functionalized to the nanoparticles and an oligonucleotide duplex linker which links the distinct oligonucleotide probes present in the surface of the nanoparticles. The duplex linker functions as the target for the bacterial nucleases, being cleaved if bacterial nucleases are present, leading to the dispersion of the nanoparticles. In the absence of bacterial nucleases, the nanoparticles aggregate.

In order to overcome the problems of previous methods known in the art, the oligonucleotide probes were carefully designed to avoid the formation of secondary structures, the duplex linker was carefully designed to be a target to bacterial nucleases, and the functionalization of the nanoparticles optimized in order to guarantee a reliable, stable and reproducible method.

Therefore, an aspect of the present invention relates to an *in vitro* method for colorimetric detection of bacterial nucleases in a food sample, here onwards the method of the invention, comprising:
a) providing a hemi-methylated oligonucleotide duplex linker with overhanging ends;
b) mixing the duplex linker of step (a) with a food sample forming a reaction solution;
c) incubating the reaction solution of step (b) for sufficient time to allow the cleavage of the duplex linker in case nucleases are present. in the reaction mixture;
d) providing at least two types of functionalized nanoparticles, wherein the first type of nanoparticle is functionalized with an oligonucleotide probe being complementary to one of the overhanging ends of the duplex linker, and the other type of nanoparticle is functionalized with a different oligonucleotide probe being complementary to the other overhanging end of the duplex linker;
e) adding the nanoparticles obtained in step d) to the reaction solution from (c), forming the final solution, and incubate under conditions effective for the hybridization of the duplex linker comprising overhanging ends with the nanoparticles oligonucleotide probes; and
f) examining the final solution obtained in step (e) wherein if the final solution colour shifts to red due to the dispersion of the nanoparticles, it indicates the presence of the bacterial nucleases in the food sample, while if the solution mixture colour shifts to blue due to the aggregation of the nanoparticles, it indicates the absence of bacterial nucleases in the food sample.

The term "colorimetric detection" refers to the detection of colour changes of a solution, due to the state of aggregation of the nanoparticles, without the aid of an intervening processing step (e.g., conversion of a colour change into an electronic signal that is processed by an interpreting device). It is intended that the term encompass visual observing (e.g., observing with the human eye). As mentioned before, due to the redshift of localised surface plasmon resonance of nanoparticles, when these come together, i.e. form aggregates, a change in the colour of the solution is visible towards blue/purple tones. Otherwise, when nanoparticles are dispersed in the solution, the colour of the solution is in red tones.

The term "bacterial nuclease" as used herein refers to naturally-occurring bacterial enzymes which cleave a phosphodiester bond within a polynucleotide chain. In a preferred embodiment of the method of the invention the bacterial nucleases are from *Salmonella spp*., *Staphylococcus* spp., *Listeria* spp., *E. coli, Campylobacter* spp., *Shigella* spp., *Yersinia* spp., *Vibrio* spp., *Mycoplasma* spp., *Legionella* spp., *Bacillus* spp., *Klebsiella* spp., *Enterococcus* spp., *Streptococcus* spp., *Clostridium* spp., *Haemophilus* spp., *Pseudomonas* spp., *Acinetobacter* spp. *Helicobacter* spp., more preferably from *S. typhimurium, S. enteritidis, S. aureus* or any combination of said bacterial nucleases.

The term "food sample" as used herein means a foodstuff or beverage. The foodstuff may be a solid or fluid. The term "food sample" includes, without limitation, natural food raw materials, synthetic food raw materials, and processed or cooked (ready-to-eat products), simple or composed foods, and the natural food raw materials include, for example, fruits, vegetables, poultry, cereals, dairy products such as cheese, milk, yoghurt, cream, bakery products, chocolate, peanut butter, beverages and the like, eggs, unprocessed meat and fish, but are not limited thereto. For convenience of explanation, examples of food samples of bread, duck, pancakes, chicken, lettuce, omelette, potato salad and cottage cheese are described (see examples), but such examples do not limit the scope of the present invention.

The term "oligonucleotide" used herein refers to short nucleic acid polymers, single or double stranded, being composed of DNA (gDNA, cDNA), RNAs (sense RNAs, antisense RNAs, mRNAs, tRNAs, rRNAs, small interfering RNAs (siRNAs), doublestranded RNAs (dsRNA), short hairpin RNAs (shRNAs), piwi-interacting RNAs (PiRNA), micro RNAs (miRNAs), small nucleolar RNAs -(SnoRNAs), small nuclear RNAs (SnRNAs), ribozymes, aptamers, DNAzymes, ribonuclease-type complexes and other such molecules as herein described. For the avoidance of doubt, the term "oligonucleotides" includes non-naturally occurring modified forms, as well as naturally occurring forms. The term "duplex" as used herein refers to a double stranded oligonucleotide. The term "duplex linker" as used herein refers to a double stranded oligonucleotide with overhanging ends, i.e. a double stranded oligonucleotide wherein the strands that compose the duplex only hybridize in a limited central region. When intact, the "duplex linker" connects, joins or attaches two distinct oligonucleotides that hybridize to the overhanging ends.

The term "hemi-methylation" or "hemimethylation" as used herein refers to the methylation state of a double stranded oligonucleotide wherein only one strand thereof presents one or more nucleotides, in any combination, 5' modified with methyl, i.e. 5-methyladenine ("5-mA"), 5-methylthymine ("5-mT"), 5-methylcytosine ("5-mC"), 5-methylguanine ("5-mG") within a DNA sequence. In a further embodiment of the method of the invention the hemi-methylated oligonucleotide duplex linker comprises two strands of RNA or one strand of DNA and one strand of RNA.

Since the hemi-methylated duplex linker has to fulfil two functions, it requires a careful design of the oligonucleotide sequence. On one hand, the duplex linker has to be able to induce aggregation of nanoparticles, while on the other hand, it has to be sensible to the nucleases. Due to this dual functionality, not only the sequence, but the types of oligonucleotides and the combination of the terminus (5'end or 3'end) of the duplex linkers are important to the performance of the method of the invention. Therefore, in a preferred embodiment of the method of the invention, the methylated strand of the hemi-methylated duplex linker comprises an oligonucleotide sequence selected from the list consisting of: SEQ ID NO: 8, SEQ ID NO: 11 and SEQ ID NO: 14; and the non-methylated strand of the duplex linker comprises an oligonucleotide sequence selected from the list consisting of: SEQ ID NO: 3, SEQ ID NO:4, SEQ ID NO:9, SEQ ID NO: 10, SEQ ID NO: 12 and SEQ ID NO: 13, preferably wherein the hemi-methylated duplex linker is composed by SEQ ID NO: 8 and SEQ ID NO: 3 or SEQ ID NO: 8 and SEQ ID NO: 4 or SEQ ID NO: 11 and SEQ ID NO: 10 or SEQ ID NO: 14 and SEQ ID NO: 13 or SEQ ID NO: 11 and SEQ ID NO: 9 or SEQ ID NO: 14 and SEQ ID NO: 12. The oligonucleotide sequences used in the present invention are shown in Table 1.

**Table 1. Oligonucleotide sequences used in the present invention.**

| **Oligonucleotides names** | **Oligonucleotide Sequence (5' > 3')** |
|---|---|
| Probe A (SEQ ID NO: 1) | *AAC-GAC-TCA-TAT-TAA-CAA |
| Probe B (SEQ I D NO: 2) | *TAG-TCT-CAT-TTA-TGC-TAT |
| Linker A DNA (SEQ ID | GTA-AGT-AGT-AGA-TTG-TTA-ATA-TGA-GTC-GTT |
| NO: 3) | |
| Linker A RNA (SEQ ID NO: 4) | GUA-AGU-AGU-AGA-UUG-UUA-AUA-UGA-GUC- GUU |
| Linker A 2'OMe (SEQ ID NO: 5) | mGmUmA-mAmGmU-mAmGmU-mAmGmA- mUmUmG-mUmUmA-mAmUmA-mUmGmA- mGmUmC-mGmUmU |
| Linker B DNA (SEQ ID NO: 6) | TCT-ACT-ACT-TAC-ATA-GCA-TAA-ATG-AGA-CTA |
| Linker B RNA (SEQ ID NO: 7) | UCU-ACU-ACU-UAC-AUA-GCA-UAA-AUG-AGA- CUA |
| Linker B 2'OMe (SEQ ID NO: 8) | mUmCmU-mAmCmU-mAmCmU-mUmAmC- mAmUmA-mGmCmA-mUmAmA-mAmUmG- mAmGmA-mCmUmA |
| Linker C DNA (SEQ ID NO: 9) | GCA-AGC-AGC-AGA-TTG-TTA-ATA-TGA-GTC- GTT |
| Linker C RNA (SEQ ID NO: 10) | GCA-AGC-AGC-AGA-UUG-UUA-AUA-UGA-GUC- GUU |
| Linker D 2'OMe (SEQ ID NO: 11) | mUmCmU-mGmCmU-mGmCmU-mUmGmC- mAmUmA-mGmCmA-mUmAmA-mAmUmG- mAmGmA-mCmUmA |
| Linker E DNA (SEQ ID NO: 12) | GAA-AGC-AGG-AGA-TTG-TTA-ATA-TGA-GTC- GTT |
| Linker E RNA (SEQ ID NO: 13) | GAA-AGC-AGG-AGA-UUG-UUA-AUA-UGA-GUC- GUU |
| Linker F 2'OMe (SEQ ID NO: 14) | mUmCmU-mCmCmU-mGmCmU-mUmUmC- mAmUmA-mGmCmA-mUmAmA-mAmUmG- mAmGmA-mCmUmA |

| | |
|---|---|
| *These sequences are modified at the 5' end with a Thiol group followed by a PEG spacer followed by the nucleotide sequence (SH-Spacer₁₈-nucleotide_sequence). | |

The "mixing the duplex linker with a food sample forming a reaction solution" in step b) of the method of the invention, relates to the process of adding the duplex linker to a solution containing the food sample to be tested for the presence of bacterial nucleases. Such addition is made to ensure the optimal concentration of the duplex linker in order to avoid false positive or negative results due to too high or too low concentration of the duplex linker in the solution. The optimal concentration of the duplex linker is related to the concentration of nanoparticles that need to be above a certain concentration threshold to ensure detection of colour changes in the solution. Too low concentration of the duplex linkers causes false positive since all linkers distribute equally among available nanoparticles at large excess without causing the aggregation regardless of the enzymatic activity of the nucleases. Too high concentrations of the duplex linkers also cause false positives since at high excess the duplex linkers saturate the available surface of the few nanoparticles regardless of the enzymatic activities of the nuclease. In a preferred embodiment of the method of the invention, the concentration of duplex linker in the reaction solution is 15 to 45 nM, preferably 25 nM to 30 nM, more preferably at least 25, 26, 27, 28, 29, 30 nM.

The term "cleavage of the duplex linker" as used herein refers to the break, cleavage, cut or degradation of a phosphodiester bond by a bacterial nuclease in the double stranded region of the duplex linker.

The "incubating the reaction solution for sufficient time to allow degradation of the duplex linker" in step c) of the method of the invention, relates to the process of allowing the bacterial nucleases, if present in the solution, to cleave the duplex linker, being that this process is allowed to occur during a sufficient amount of time and at an ideal temperature for the optimal function of the bacterial nuclease to avoid false negatives due to too little time of incubation and/or non-ideal temperature for the bacterial nucleases. In a preferred embodiment of the method of the invention, step c) is performed at 20 to 45°C during 20 to 110 min, preferably at 30 to 40°C during 15 to 45 min, more preferably at 37°C during 30 min. Longer incubation times suffer from poor selectivity, while shorter incubation times affect sensitivity since not enough time is given for the bacterial nucleases to cleave the duplex linker. The term "selectivity" as used herein refers to the ability of the duplex linker used in the method of the invention to be cleaved only by the nuclease which it targets. The term "sensitivity" as used herein refers to the ability of the method of the invention to provide a positive result when a bacterial nuclease is present in a sample.

The term "nanoparticle", as used herein, refers to an ultrafine particle of spherical, cube-like, rod-like, bipyramidal, or prism-like shape, between 2 and 200 nm in size that is formed by gold or silver atoms. In a preferred embodiment of the method of the invention, the nanoparticles are gold or silver nanoparticles, preferably gold nanoparticles, and their morphology is spherical, cube-like, rod-like, bipyramidal, or prism-like, preferably spherical. In another preferred embodiment the shape. nanoparticles size is between 15 to 50 nm, preferably between 22 to 30 nm, more preferably at least 22, 23, 24, 25, 26, 27, 28, 29 and 30 nm. Smaller nanoparticles (<15nm) harmed the stability of the method of the invention, which could lead to false positive results, and thus, lack of specificity, while bigger nanoparticles (>50nm) tend to aggregate non-specifically under high ionic strength conditions in the reaction solution, giving false negative results and therefor reducing sensitivity.

Nanoparticles suitable for the present invention are nanoparticles obtained by a simple method of synthesis, having a narrow size distribution and capable of being easily conjugated to single stranded DNA. As non-limiting examples of the production of nanoparticles suitable for the method of the invention, gold nanoparticles are produced in a liquid by reduction of chloroauric acid by sodium citrate (Bastús et al., Langmuir 2011, 27 (17), 11098-11105), while silver nanoparticles are produced by reduction of silver ion (Ag⁺) in aqueous solution, receiving an electron from a reducing agent to switch from a positive valence into a zero-valent state (Ag0), followed by nucleation and growth (Lee and Jun, Int J Mol Sci. 2019 Feb; 20(4): 865, 1-24). Different methods are known by the person skilled in the art to manufacture nanoparticles.

The term "functionalized nanoparticle," as used herein, refers to a nanoparticle having at least a portion of its surface modified with an oligonucleotide, the oligonucleotide probe. The term "oligonucleotide probe" as used herein, refers to the oligonucleotide attached to the surface of the nanoparticle which is complementary and can hybridize with one of the overhanging ends of the duplex linker. The nanoparticle surface needs to carry a minimum number of oligonucleotide probes to ensure colloidal stability of the nanoparticle, preferably at high salt concentrations (long enough shelf lifetime) and to prevent unspecific aggregation that could lead to false negative (low sensitivity) results. Thus, in a preferred embodiment of the method of the invention, the nanoparticles are functionalized with 40 to 140 probes in their surface, preferably 50 to 60 probes.

In a preferred embodiment of the method of the invention, the oligonucleotide probes content of GC is less than 30%. The term "content of GC" as used herein refers to the percentage of nitrogenous bases in a DNA or RNA molecule that are either guanine (G) or cytosine (C). This measure indicates the proportion of G and C bases out of an implied four total bases, also including adenine and thymine in DNA and adenine and uracil in RNA. The oligonucleotide probes are designed with less than 30% of GC content in order to minimize the probability of the formation of any secondary structures and self-complementary hybridization of the probes in order to keep the detection time of the method of the invention to a minimum.

In a further preferred embodiment of the method of the invention, oligonucleotide probes comprise an oligonucleotide sequence selected from a list consisting of: SEQ ID NO: 1 and SEQ ID NO: 2 (Table 1). The oligonucleotide probes are designed to allow a faster hybridization of the oligonucleotide duplex, shortening the reaction time of the assay and guaranteeing the colloidal stability of the nanoparticles, preferably gold nanoparticles, even in high ionic strength media, minimizing the risk of false negative results.

In a preferred embodiment of the method of the invention, the oligonucleotide probes can be modified at their extremities, preferably at their 5' end, more preferably with a thiol group. The term "thiol" as used herein refers to an organosulfur compound of the form R-SH, where R represents an alkyl or other organic substituent, S represents a sulphur atom and H and hydrogen atom. In a further preferred embodiment of the method of the invention, the oligonucleotide probes comprise a spacer of 18 poly-ethylene-glycol (PEG₁₈) groups, according to the structure H-[O-CH₂-CH₂]₁₈-OH, located between the thiol group at the 5'end and the nucleotide sequence of the oligonucleotide probe. The presence of a PEG₁₈ spacer improves colloidal stability of the nanoparticle, as compared to other types of spacers: 10T or 10A bases, commonly used in the field. The PEG₁₈ spacer increases the flexibility of the capture probes strain on the nanoparticles surface, increasing the hybridization rate which translates to a shorter detection time of the method of the invention.

In a preferred embodiment of the method of the invention, the ratio of linkers and nanoparticles is 100 to 200, preferably 150 to 175, more preferably at least 150, 151, 152, 153, 154, 155, 156, 157, 158, 159, 160, 161, 162, 163, 164, 165, 166, 167, 168, 169, 170, 171, 172, 173, 174, 175. The term "ratio" as used herein refers to the molar concentration of linkers and functionalized nanoparticle.

The "incubate under conditions effective for the hybridization of the duplex linker comprising overhanging ends with the nanoparticles oligonucleotide probes" in step e) of the method of the invention, relates to the process of allowing the functionalized nanoparticles to interact and hybridize with the duplex linker, leading to the formation, or absence in case bacterial nucleases are present, of aggregates. In order for this process to occur sufficient time must be allowed for the hybridization to occur. In a preferable embodiment the incubation time is 20 to 110 min, preferably 30 to 60 min, more preferably at least 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 min.

Also important for this process is the salt concentration in the final solution. At low salt concentration, the specificity of the method of the invention is usually high, but longer detection times are required. At high salt concentration, fast detection is possible at the expense of specificity. The salt concentration can affect duplex linker dimerization and in consequence particles hybridisation. Note that the duplex linkers contain different subunits (DNA vs. 2'OMe) and (RNA vs. 2'OMe) in which the differences in the electrostatic charges along the nucleic acid chains can alter H bonding. An example of a salt suitable for the reaction solution is sodium chloride. Other salts can be used and the expert in the art will be able to determine a suitable salt to use in the reaction solution. In a preferred embodiment of the method of the invention, step (e) is carried out in the presence of salt at a concentration in the final solution of 0.1 to 0.3 M, preferably 0.2 M, more preferably 0.208 M wherein the duplex linkers comprise RNA and 2-OMe, yet more preferably 0.278 M wherein the duplex linkers comprise DNA and 2-OMe.

The term "salt(s)" as used herein refers to a chemical compound consisting of an ionic assembly of cations and anions. Salts are composed of related numbers of cations (positively charged ions) and anions (negatively charged ions) so that the product is electrically neutral (without a net charge). These component ions can be inorganic, such as chloride (Cl⁻), or organic, such as acetate (CH₃CO₂⁻); and can be monatomic, such as fluoride (F⁻) or polyatomic, such as sulphate (SO₄²⁻). In the present invention, the preferred salts are inorganic salts.

The presence of calcium (Ca²⁺) in the reaction mixture is also important for the process, since this ion is required for the activity of the nucleases and in addition it permits the increase in the signal to noise ratio of the method of the invention. In a preferred embodiment, step (c) is carried out in the presence of calcium at a concentration of up to 20 mg/L in the reaction mixture, preferably 1 to 5 mg/L, more preferably at least 1, 2, 3, 4, 5 mg/L.

A further aspect of the invention relates to a kit for colorimetric detection of bacterial nucleases, here onwards the kit of the invention, comprising a least one hemi-methylated oligonucleotide duplex linker with overhanging ends and at least two types of functionalized nanoparticles, each type functionalized with a different oligonucleotide probe, wherein the hemi-methylated oligonucleotide duplex linker in the kit comprises a methylated strand, preferably selected from a list consisting of: SEQ ID NO: 5, SEQ ID NO: 8, SEQ ID NO: 11 and SEQ ID NO: 14; and a non-methylated strand of the duplex linker comprises an oligonucleotide sequence, preferably selected from the list consisting of: SEQ ID NO: 3, SEQ ID NO:4, SEQ ID NO: 6, SEQ ID NO: 7, SEQ ID NO:9, SEQ ID NO: 10, SEQ ID NO: 12 and SEQ ID NO: 13, and the oligonucleotide probe sequence is preferably selected from the list consisting of: SEQ ID NO 1 and SEQ ID NO: 2, preferably wherein the hemi-methylated duplex linker is composed by SEQ ID NO: 8 and SEQ ID NO: 3 or SEQ ID NO: 8 and SEQ ID NO: 4 or SEQ ID NO: 11 and SEQ ID NO: 10 or SEQ ID NO: 14 and SEQ ID NO: 13 or SEQ ID NO: 11 and SEQ ID NO: 9 or SEQ ID NO: 14 and SEQ ID NO: 12.

The kit may further include other components that find use in the subject invention, e.g., buffers, delivery vehicles, positive and/or negative controls components optionally wherein such controls comprise oligonucleotide duplexes, containers with solutions or empty that encounter use in the practicing of the subject invention. In a preferred embodiment the kit of the invention further comprises a negative control formed by fully methylated oligonucleotide duplex with overhanging ends, preferably the oligonucleotide duplex comprising SEQ ID NO: 5 and SEQ ID NO: 8, since 2'OMe modification in both strands of the duplex linker (SEQ ID NO: 5 and 8) prevents duplex linker fragmentation.

The various components of the kit may be present in separate containers or certain compatible components may be combined into a single container, as desired. In addition to the above components, the subject kits will further include instructions for practicing the subject invention. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g. a CD, an USB, etc., on which the information has been recorded. Yet another means that may be present is a website address which may be used via the internet to access the information at a removed site. Any convenient means may be present in the kits.

Another aspect of the invention is the use *in vitro* of the kit of the invention for colorimetric detection of bacterial nucleases according to the method of the invention.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### DESCRIPTION OF THE DRAWINGS

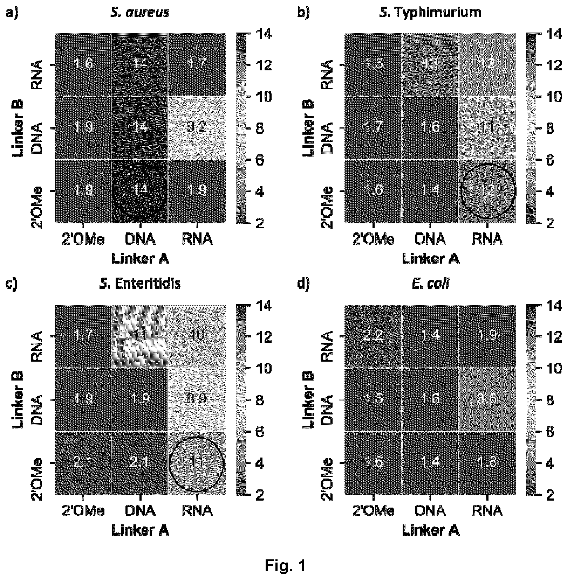

**Fig. 1****. Selection of duplex linker composition.** Screening of different modifications of duplex linkers A and B (Table 1) after incubation with sterile bacterial supernatants of **(a)** *Staphylococcus aureus* strain 1, **(b)** *Salmonella* Typhimurium ATCC 14028, **(c)** *Salmonella* Enteritidis ATCC 49214 and **(d)** *Escherichia coli* ATCC 25981 cultures. For each duplex linker combination, dispersion degree D values are highlighted according to the outcome from colorimetric discrimination (threshold of positivity at D>5; see Materials and Methods). Circles inside squares indicate the duplex linker composition selected for further experiments.

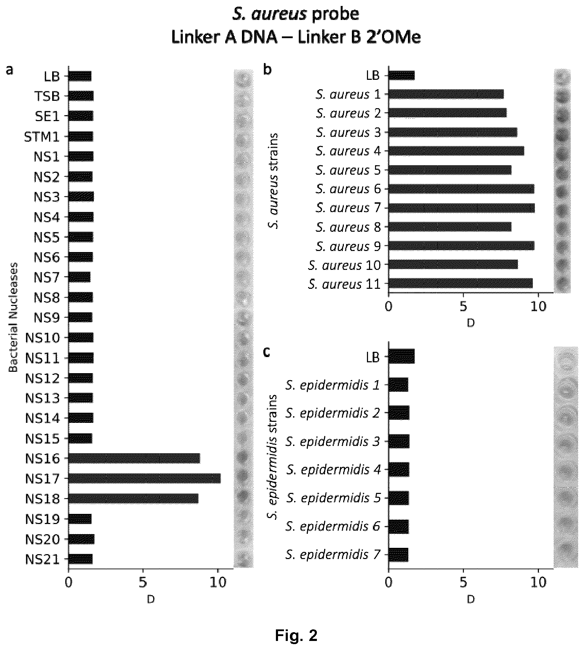

**Fig. 2****. Selective discrimination of *S. aureus* from other bacteria nucleases by using the method of the invention.** The duplex linker comprising DNA linker A SEQ ID NO: 3 and 2'OMe linker B SEQ ID NO: 8 (Table 1) allows the specific detection of **(b)** *S. aureus* (n=11), discriminating it from **(c)** *S. epidermidis* (n=7) as well as **(a)** *Salmonella* (strains SE1, STM1; Table 2) and other Non-Staphylococcal bacteria (NS1 to NS21; Table 3). Each quantitative bar represents the dispersion degree value (threshold of positivity at D>5; see Materials and Methods) and is accompanied by a naked eye digital image of a plastic-well containing the assay mixture.

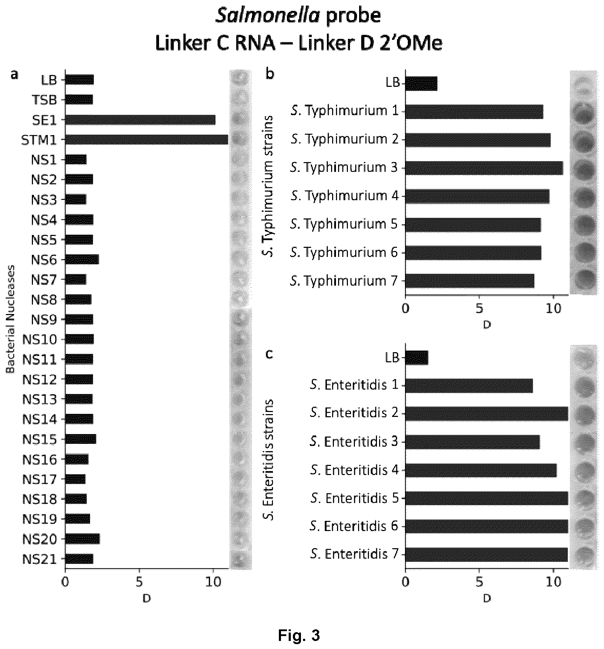

**Fig. 3****. Selective discrimination of *Salmonella* from other bacterial nucleases, by using the method of the invention.** The duplex linker comprising RNA linker C SEQ ID NO: 10 and 2'OMe linker D SEQ ID NO: 11 (Table 1) allows the specific detection of both **(b)** *S.* Typhimurium and **(c)** *S.* Enteritidis strains (n=7; Table 2) as well as **(a)** to discriminate *Salmonella* from *Non-Salmonella* bacteria (strains NS1 to NS21; Table 3). Each quantitative bar represents the dispersion degree value (threshold of positivity at D > 5; see Materials and Methods) and is accompanied by a naked eye digital image of a plastic-well containing the assay mixture.

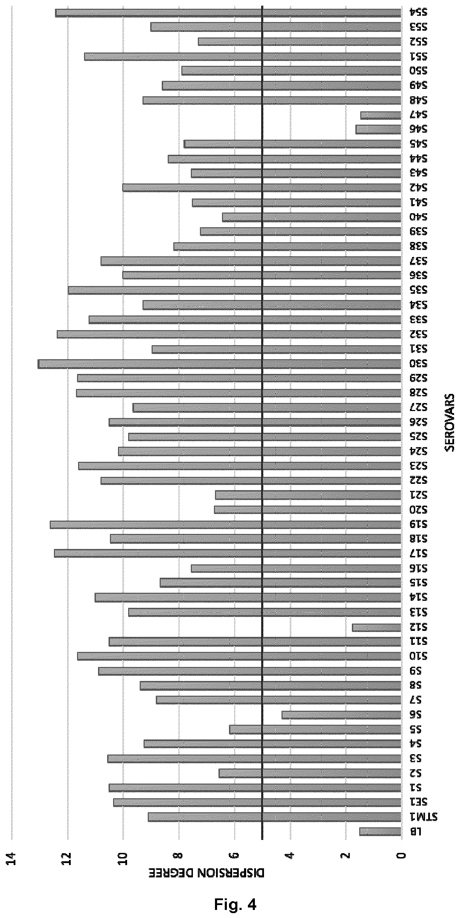

**Fig. 4****. Detection of different *Salmonella* species and serovars, by using the method of the invention.** The duplex linker comprising RNA linker C SEQ ID NO: 10 and 2'OMe linker D SEQ ID NO: 11 (Table 1) detected 52/56 different *Salmonella* species and serovars analysed (Table 2). Each quantitative bar represents the dispersion degree value (threshold of positivity at D > 5; see Materials and Methods).

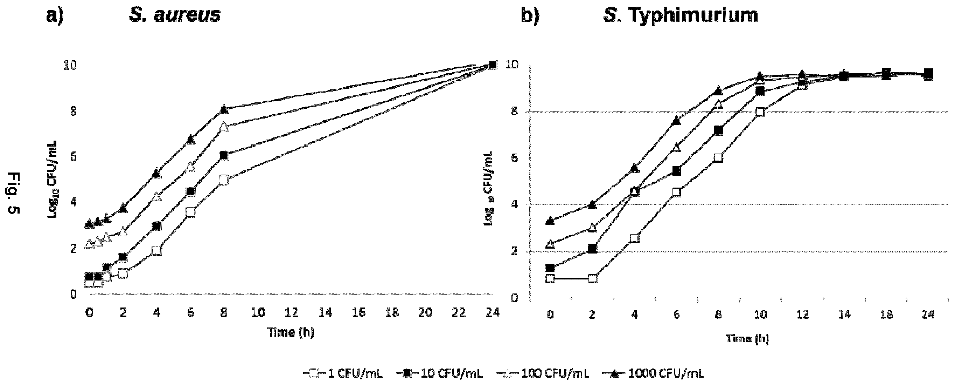

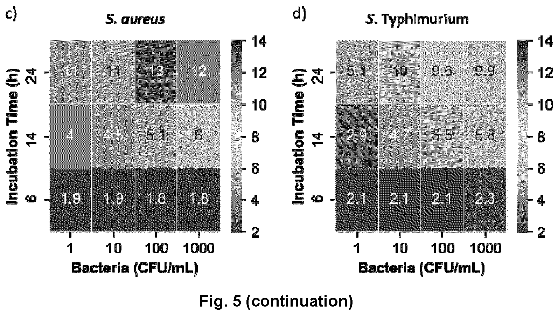

**Fig. 5****. *S. aureus* and *S.* Typhimurium growth curves *in vitro* and limit of detection of nucleases in the supernatants, by using the method of the invention.** Different concentrations ranking from 10° to 10³ CFU/mL of *S. aureus* strain 1 and *S.* Typhimurium ATCC 14028 cultures were adjusted by spectrophotometry in fresh Buffer Peptone Water (BPW) and analysed for both **(a, b)** bacterial counting (log₁₀ CFU/mL) and **(c, d)** nucleases detection, at different intervals of incubation (37°C) from 0 to 24h. Sterile bacterial supernatants obtained at 6, 14 and 24 h of incubation intervals were mixed with the duplex linkers DNA linker A SEQ ID NO: 3 and 2'OMe linker B SEQ ID NO: 8 (Table 1) for *S. aureus* detection **(c)** or RNA linker C SEQ ID NO: 10 and 2'OMe linker D SEQ ID NO: 11 (Table 1) for *S.* Typhimurium detection **(d).** Each quantitative square represents the dispersion degree value (threshold of positivity at D>5; see Materials and M**ethods).**

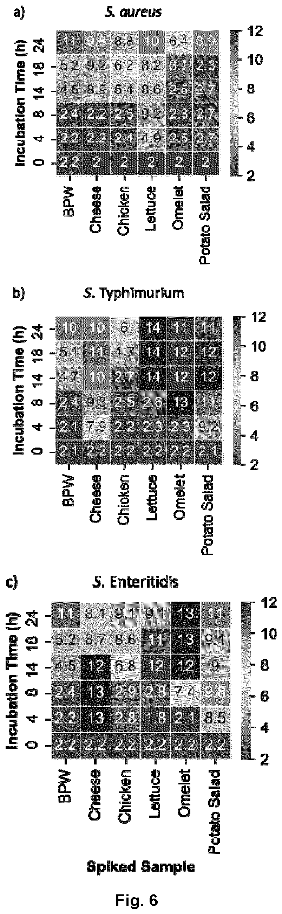

**Fig. 6****. Detection of bacterial nucleases in spiked food samples experimentally contaminated with (a) *S. aureus,* (b) *S.* Typhimurium or (c) *S.* Enteritidis, at different incubation intervals.** Cheese, chicken, lettuce, omelette or potato salad samples were contaminated with 10³ CFU of **(a)** *S. aureus* 1, **(b)** *S.* Typhimurium ATCC 14028 and **(c)** *S.* Enteritidis ATCC 49214 strains and the supernatants of each culture were obtained at 0, 4, 8, 14, 18 and 24 h post-incubation (37°C). BPW medium was used as bacteria-free control. After filtration, sterile supernatants were incubated with the duplex linkers composed by DNA linker A SEQ ID NO: 3 and 2'OMe linker B SEQ ID NO: 8 (Table 1) for the detection of *S. aureus* or with RNA linker C SEQ ID NO: 10 and 2'OMe linker D SEQ ID NO: 11 (Table 1) for the detection of *S.* Typhimurium and *S.* Enteritidis (Table 2). Results are expressed as the dispersion degree value (threshold of positivity at D>5; see Materials and Methods).

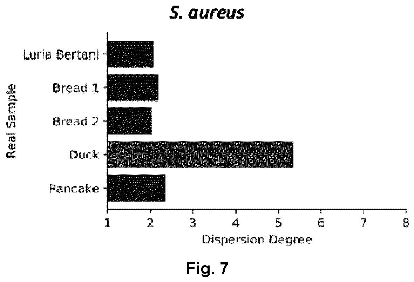

**Fig. 7****. Detection of *S. aureus* nucleases in ready-to-eat products, by using the method of the invention.** *S. aureus* nucleases were detected in duck naturally contaminated with the pathogen. Bread and pancake products resulted naturally contaminated with *S. epidermidis.*

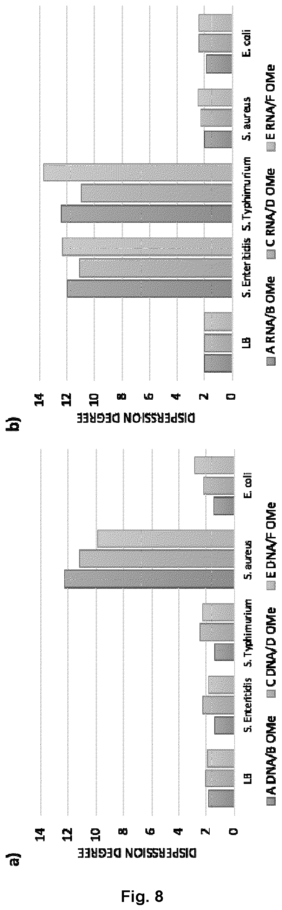

**Fig. 8****. Colorimetric detection of *Staphylococcus aureus* and *Salmonella* nucleases, using different sets of duplex linkers.** Three different combinations of duplex linkers were used to detect **(a)** *S. aureus* and **(b)** *S.* Typhimurium and *S.* Enteritidis nucleases. The duplex linkers were: **(a)** DNA linker A SEQ ID NO: 3 and 2'OMe linker B SEQ ID NO: 8; DNA linker C SEQ ID NO: 9 and 2'OMe linker D SEQ ID NO: 11; and DNA linker E SEQ ID NO: 12 and 2'OMe linker F SEQ ID NO: 14, for *S. aureus;* and **(b)** RNA linker A SEQ ID NO: 4 and 2'OMe linker B SEQ ID NO: 8; RNA linker C SEQ ID NO: 10 and 2'OMe linker D SEQ ID NO: 11; RNA linker E SEQ ID NO: 13 and 2'OMe linker F SEQ ID NO: 14, for *Salmonella.* Oligonucleotide sequences used are shown in Table 1. Results are expressed as the dispersion degree value (threshold of positivity at D>5; see Materials and Methods).

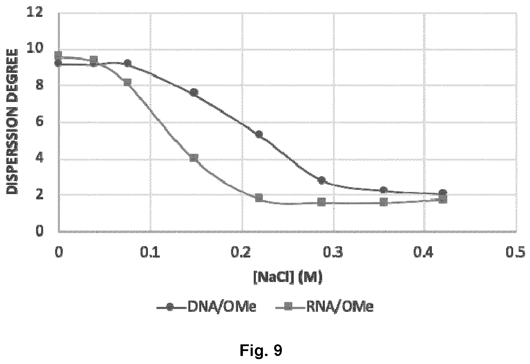

**Fig. 9****. Influence of NaCl concentration in the dispersion degree of the method of the invention.** *S. aureus* sterile supernatants were mixed for 20 minutes with two different duplex linkers combination: DNA linker A SEQ ID NO: 3 and 2'OMe linker B SEQ ID NO: 8 (square symbols) or RNA linker C SEQ ID NO: 10 and 2'OMe linker D SEQ ID NO: 11 (circle symbols). Oligonucleotide sequences used are shown in Table 1. Results are expressed as the dispersion degree value (threshold of positivity at D>5; see Materials and Methods).

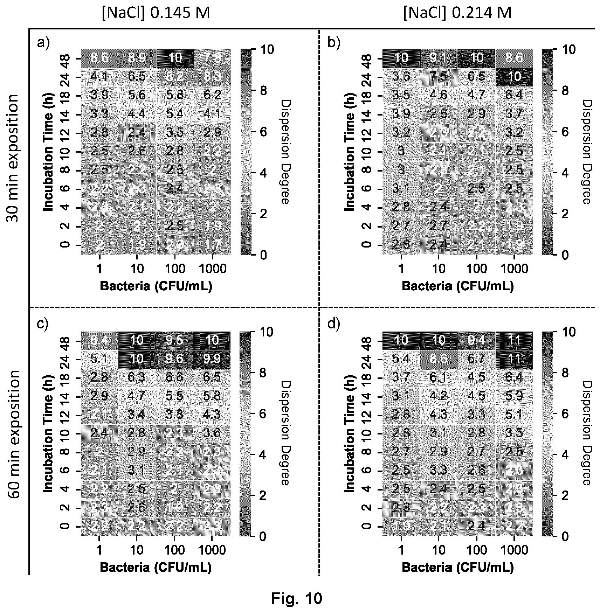

**Fig. 10****. Optimization of detection parameters of the method of the invention.** Combination of different NaCl concentration (0.145 M and 0.214M) and exposition time (30 min and 60 min), for *in vitro S.* Typhimurium ATCC 14028 supernatant samples. The duplex linker used was composed by RNA linker C SEQ ID NO: 10 and 2'OMe linker D SEQ ID NO: 11 (Table 1). Results are expressed as the dispersion degree value (threshold of positivity at D>5; see Materials and Methods).

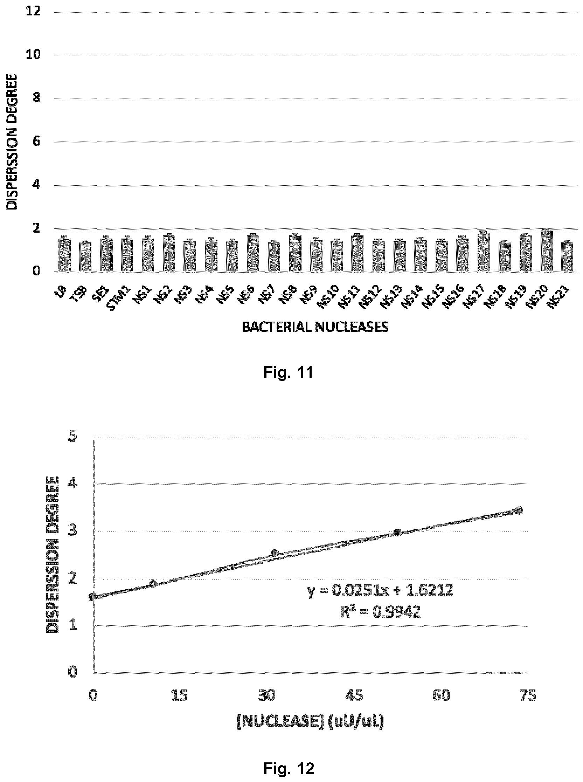

**Fig. 11****. Example of inadequate combination of linkers for the method of the invention.** Sterile supernatants from *S. aureus, S.* Typhimurium (Table 2) and bacterial contaminants (Table 3) were mixed with 2'OMe linker A SEQ ID NO: 5 and 2'OMe linker B SEQ ID NO: 8 (Table 1) giving negative results. Results are expressed as the dispersion degree value (threshold of positivity at D>5; see Materials and Methods).
**Fig. 12****. Calibration curve for determining the limit of detection of nucleases.** Increasing concentrations of a commercial micrococcal nuclease were tested with a mix of DNA linker A SEQ ID NO: 3 and 2'OMe linker B SEQ ID NO: 8 (Table 1). Results are expressed as the dispersion degree value (see Materials and Methods) obtained for each nuclease concentration. Statistical regression analysis provides the mathematical parameters (i.e. intercept, slope, R-square and adjusted R-square).

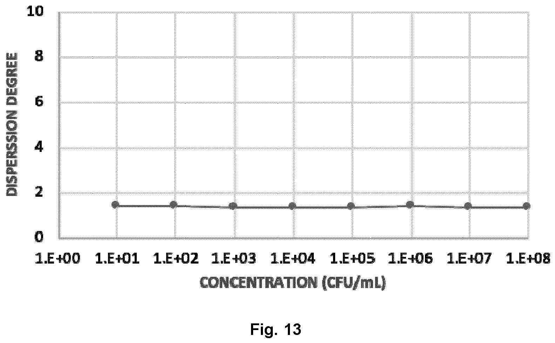

**Fig. 13****. Bacterial concentration does not correlate with the presence of detectable nucleases.** Supernatants of *S. aureus* suspensions carrying different bacterial concentrations (i.e. 10¹ to 10⁸ CFU/mL) were generated and immediately , without previous bacterial incubation, measured by using the method of the present invention. Bacterial concentrations of each inoculum were retrospectively assessed by serial dilutions and plating (37°C, 24h). Sterile supernatants were incubated with DNA linker A SEQ ID NO: 3 and 2'OMe linker B SEQ ID NO: 8 (Table 1) and the dispersion degree was measured (threshold of positivity at D>5; see Materials and Methods).

### Examples

### Materials and Methods

**Chemicals.** Hydrogen tetrachloroaurate (III) trihydrate (HAuCl₄·3H₂O) was purchased from Alfa Aesar. Sodium dodecyl sulfate (SDS) (98%), sodium chloride (NaCl) (99.5%), sodium citrate tribasic dihydrate (98%), and phosphate buffer (PB) 1 M, pH 7.4, were purchased from Sigma-Aldrich. Phosphate buffered saline (PBS) 0.01 M, pH 7.4, containing 0.138 M NaCl and 2.7 mM KCI (Sigma-Aldrich) was used to mimic physiological conditions. Two types of Phosphate Buffered Saline (PBS, pH 7.4) with MgCl2 and CaCl2 (PBS +/+) and without this supplement (PBS -/-) were purchase from Gibco (Life Technologies, Spain). Luria Bertani (LB) and Tryptic Soy Broth (TSB) culture media were purchase in Difco (Pronadisa, Spain). DNA sequences and thiolated-oligonucleotides were purchased from Biomers (Germany). Commercial micrococcal nuclease was purchased from Thermofisher.

**Instrumentation.** UV-Vis-NIR spectra were measured at room temperature on a Jasco UV-Vis spectrophotometer, using UV Micro cuvettes with 1 cm optical path length. Transmission electron microscopy (TEM) was measured in a JEOL JEM-1400 PLUS, operating at 120 kV. Dynamic light scattering (DLS) measurements were carried out in a Malvern NanoSizer. A Veriti Thermal Cycler 60-well Applied Biosystems was used for the incubation of the samples.

**Synthesis and functionalization of 30 nm gold nanoparticles (hereafter AuNPs).** AuNPs were synthesized following a seeded growth method (Bastús et al., Langmuir 2011, 27 (17), 11098-11105). The particles were then functionalized with thiolated oligonucleotide probe A (SEQ ID NO: 1) or probe B (SEQ ID NO: 2) according to the method reported previously (Hurst, S. J.; Lytton-Jean, A. K. R.; Mirkin, C. A. Anal. Chem. 2006, 78 (24), 8313-8318). Briefly, to the AuNPs colloid (5.55 mL) containing SDS (0.1%) and Pb (0.01 M) was added a solution of oligonucleotide probe A (SEQ ID NO: 1) or a solution of oligonucleotide probe B (SEQ ID NO: 2) (0.1 mM). An excess of oligonucleotides was used in all samples. The mixture of oligonucleotides and AuNPs was incubated at room temperature for 20 min. To improve oligonucleotide binding, a salt aging process was carried out. A solution containing NaCl (2 M), SDS (0.01%), and PB (0.01 M) was added sequentially to the mixture containing AuNPs and oligonucleotides, in the following aliquots: 25, 25, 75, 125, and 250 µL, reaching a final NaCl concentration of 0.2 M. Each salt aging step was alternated with sonication (10 s) and incubation (20 min), followed by incubation for 12 h. To remove excess oligonucleotides, the solutions were centrifuged (6,500 rpm, 30 min, 3 times) and each time redispersed in SDS (4 mL, 0.01%). The final concentration of nanoparticles was 0.6 mM in terms of Au atoms for all samples.

**Detection of bacterial nucleases.** 7 µL of sample (culture supernatant) were combined with 1µL of PBS (+/+) and 1 µL (10 µM) of each oligonucleotide that composes the duplex linkers (Table 1) (nuclease target). This mixture was incubated at 37°C for 30 min. After that, 225 µL of milli Q water was added, 50 uL of each type of functionalized nanoparticles (AuNP functionalized with oligonucleotide probe A (SEQ ID NO: 1; Table 1) and AuNP functionalized with oligonucleotide probe B (SEQ ID NO: 2; Table 1), 0.6 mM in terms of AuNP), and 20 µL of NaCl 5 M in the case of DNA probes, and 15 µL of NaCl 5 M and 230 µL of milli-Q water in the case of RNA probes. 30 minutes later, this solution was added into a UV mikro-cuvette and UV-Vis measurements were performed.

**Dynamic Light Scattering (DLS) Measurements.** The conditions for DLS experiments were: 6 measurements with 5 runs of 5 s each. The study was carried out at 25°C with an equilibration time of the sample of 120 s.

### Quantification of the method of the invention

To quantify the performance of the method of the invention, we processed the UV-Vis-NIR spectra recorded after 20-30 min of exposition time, by expressing a unitless value of dispersion degree, D = AbS₅₃₀/AbS₇₀₀. To ensure a safe regime of a colorimetric detection for positive and negative outcomes, we assigned 5 < D < 14 for positive, while D < 3 for negative readout.

### Example 1 - Working principle and test of the method of the invention

The working principle of the method of the invention is based on the aggregation of two type of nanoparticles functionalized with the oligonucleotide probes in the presence of a duplex linker containing overhanging ends (Table 1), which are complementary to both types of the oligonucleotide probes functionalized to the nanoparticles (see Table 1 for sequence compositions). To exemplify the method of the invention, gold nanoparticles functionalized with oligonucleotide probes were used (here onwards abbreviated as Au@DNA) even though the results are equally valid for silver nanoparticles. In the first place, the duplex linker is exposed to the supernatant from microbial culture for 30 minutes at 37 °C, followed by the addition of Au@DNA to the solution. If the sample supernatant contains specific nuclease, the duplex linker undergoes fragmentation, inhibiting aggregation of the nanoparticles (positive test = presence of bacterial nucleases in the test sample). On the contrary, in the absence of specific nuclease, the duplex linker joins the two types Au@DNA causing gradual aggregation over 20 minutes (negative test = absence of bacterial nucleases in the test sample). Importantly, the readout of the method of the invention can be performed through eye inspection by an untrained person. To quantify the performance of the method of the invention, we also measure the dispersion degree D (see Materials and Methods).

The duplex linkers oligonucleotides (Table 1) were designed in a way to contain in total 30 bases of which 12 are complementary one to each other while remaining 18 bases are complementary to DNA immobilised on the nanoparticles surface. As a coarse screening of probe susceptibility to nucleases, we used DNA, RNA and 2'-O-Methyl (2'OMe) modified sequences (Table 1).

We evaluated all possible combinations of linker A and B (9 in total, Table 1) against four different culture supernatants: *S. aureus*, *S.* Typhimurium, *S.* Enteritidis, and *E. coli.* As a negative control, we used Luria Bertani media (LB) in all the experiments. Fig. 1 displays the values of dispersion degrees for positive (D>5) and negative (D<3) readout. The first conclusion from these data is that the presence of DNA oligonucleotides (SEQ ID NO: 3) in the duplex linker showed clear preferences for nucleases from *S. aureus,* while duplex linkers comprising RNA oligonucleotides (SEQ ID NO: 4) showed preferences for nucleases from *S.* Typhimurium and *S.* Enteritidis. In addition, we observed that 2'OMe modification in both strands of the duplex linker (SEQ ID NO: 5 and 8) prevented duplex linker fragmentation, being a suitable candidate for negative control. An interesting observation is that selectivity toward *S. aureus* and *S.* Typhimurium was maintained after replacing nucleotides but keeping 2'OMe modifications in linker B. We selected pairwise linkers combinations (black circle in Fig. 1) and substituted thymines in linker A DNA (SEQ ID NO: 3) by cytosines obtaining the sequence of linker C DNA (SEQ ID NO: 9) and the corresponding RNA sequence for linker C RNA (SE ID NO: 10), while the substitution of 2 cytosines in linker A DNA (SEQ ID NO: 3) by an adenine and a guanine originated the sequence for linker E DNA (SEQ ID NO: 12) and linker E RNA (SEQ ID NO: 13). We observed that altered sequences maintained similar selectivity toward nucleases from both *S. aureus* and *S.* Typhimurium (Fig. 8), suggesting that in our case the activities of the bacterial nucleases are sensitive to the oligonucleotide modification rather than to the sequence composition. For the next experiments, we selected the following pairwise duplex linker composition shown in Table 1: linker A DNA (SEQ ID NO: 3) and linker B 2'OMe (SEQ ID NO: 8) to detect *S. aureus*; and linker C RNA (SEQ ID NO: 10) and linker D 2'OMe (SEQ ID NO: 11) to detect *S.* Typhimurium and *S.* Enteritidis.

Apart from the type of oligonucleotide, the combination of the terminus (5'end or 3'end) of the linkers is important to the performance of the assay. For example, for *S. aureus*, swapping the linkers combination from A DNA and B 2'OMe to A 2'OMe and B DNA alters the performance of the assay.

The main drawback of colloidal biosensors based on oligonucleotides is the risk for nonspecific aggregation due to interfering species in the medium (e.g., salt concentration), which would cause a false negative in the present case. Put differently, the optimal salt concentration determines the balance between specificity and detection time. At low salt concentration, the specificity is usually high, but longer detection times are required. At high salt concentration, fast detection is possible at the expense of specificity. In our design, since we used a flexible PEG spacer in capture probes, the risk of non-specific aggregation of nanoparticles can be ruled out. However, the salt concentration can affect probe dimerization and in consequence particles hybridisation. Note that our probes contain different subunits (DNA vs. 2'OMe) and (RNA vs. 2'OMe) in which the differences in the electrostatic charges along the nucleic acid chains can alter H bonding.

We performed a salt titration of both duplex linkers, linker A DNA (SEQ ID NO: 3) or linker C RNA (SEQ ID NO: 10) in combination with linker B 2'OMe (SEQ ID NO: 8) and linker D 2'OMe (SEQ ID NO: 11), in the presence of both types of Au@DNA, finding that the optimal salt concentration ranges between 0.1 and 0.3 M (Fig. 9). We kept this salt concentration range in the following experiments. Note, however, that in the *Salmonella* probes that contain RNA and 2'OMe linkers, a lower amount (0.208 M) of salt was needed to achieve D = 5, as compared to *S. aureus* probe (0.278 M). These results suggest that RNA sequence is more sensitive to charge screening as compared to DNA.

Also, using S. Typhimurium as a model system we found that exposition time of 30 minutes is sufficient to obtain a readout of a reasonable quality (Fig. 10).

Next, we tested the specificity of the method of the invention for *S. aureus* and *Salmonella* using 23 supernatants from 15 different bacterial culture (Table 2 and Table 3). As a reference, Luria Bertani and Trypticase Soy Broth were used. All duplex linkers were incubated with supernatants at 37 °C for 30 min followed by the addition of Au@DNA and a readout (visual inspection, and D value estimation) after 30 min. As expected, the duplex linker comprising linker A DNA (SEQ ID NO: 3) and linker B 2'OMe (SEQ ID NO: 8) gave positive readout only for *S. aureus* (Fig. 2a). The same duplex linker showed excellent specificity toward 11 other strains of *S. aureus* (Fig. 2b) and negative results for 7 strains of *S. epidermidis* (Fig. 2c). Similar specificity of the method of the invention toward *S.* Typhimurium and *S.* Enteritidis against different bacterial culture (Fig. 3a) and other *S.* Typhimurium and *S.* Enteritidis strains (Fig. 3b, c) were observed using the duplex linker comprising linker C RNA (SEQ ID NO: 10) and linker D 2'OMe (SEQ ID NO: 11). In the case of the detection of *Salmonella,* 4 subspecies and 50 different serovars were tested to check the selectivity of the method (Fig. 4). Only 4 serovars produced a negative result of the assay: *S.* Coeln, *S.* Wien, *S.* Amsterdam and 6,7:-:1,5 (strains S6, S12, S46 and S47, respectively; Table 2).

**Table 2. List of Salmonella strains from ATCC or fattening pigs field isolates used in this work.**

| **Code** | ***Salmonella* serovar** | **Code** | ***Salmonella* serovar** |
|---|---|---|---|
| S1 | *enterica* subesp. *arizonae* | | |
| S2 | *enterica* subesp. *diarizonae* | | |
| S3 | *enterica* subesp. *salamae enterica* subesp. *enterica* serovar: | | |
| STM1 | *S*. Typhimurium ATCC 14028 | S28 | *S*. Mikawasima |
| STM2 to STM7 | *S*. Typhimurium | S29 | *S*. Mishmarhaek |
| SE1 | *S*. Enteritidis ATCC 49214 | 530 | *S*. Montevideo |
| 5E2 to 5E7 | *S*. Enteritidis | 531 | *S*. Muenchen |
| S4 | *S*. Abaetetuba | S32 | *S*. Newport |
| S5 | *S*. Agona | S33 | *S*. Nottingham |
| S6 | *S*. Amsterdam | S34 | *S*. Ohio |
| S7 | *S*. Bardo | S35 | *S*. Oranienburg |
| S8 | *S*. Bovismobificans | S36 | *S*. Poona |
| S9 | *S*. Braenderup | S37 | *S*. Reading |
| S10 | *S*. Branderburg | S38 | *S*. Rissen |
| S11 | *S*. Bredeney | S39 | *S*. Szentes |
| S12 | *S*. Coeln | S40 | *S*. Tennessee |
| S13 | *S*. Derby | S41 | *S*. Thompson |
| S14 | *S*. Gaminara | S42 | *S*. monophasic |
| S15 | *S*. Give | S43 | *S*. Toulon |
| S16 | *S*. Goldcoast | S44 | *S*. Umbilo |
| S17 | *S*. Hadar | S45 | *S.* Urbana |
| S18 | *S*. Havana | S46 | *S*. Wien |
| S19 | *S*. Infantis | S47 | 6,7:-:1,5 |
| S20 | *S*. Indiana | S48 | *S*. Gallinarum |
| S21 | *S*. Kapemba | S49 | *S*. Altona |
| S22 | *S*. Kedogou | S50 | *S*. Anatum |
| S23 | *S*. Kottbus | S51 | *S*. Heidelberg |
| S24 | *S*. Lexington | S52 | *S*. Muenster |
| S25 | *S*. Livingstone | S53 | *S*. Paratyphi |
| S26 | *S*. London | S54 | *S*. Strourtbridge |
| S27 | *S*. Meleagridis | | |

Interestingly, we found that a duplex linker containing 2'OMe modification in both strands, linker A 2'OMe (SEQ ID NO: 5) and linker B 2'OMe (SEQ ID NO: 8) gave negative outcome for an entire set of samples (Fig. 11), suggesting such linker combination as a negative control in practical application.

Since the method of the invention measures the bacterial activity indirectly through nucleases activity, i.e. the presence of bacterial metabolite rather whole bacterial culture, it is useful to define the minimum incubation time of given bacteria culture to obtain positive readout. For that, we tested the presence of nucleases in supernatants after 6, 14 and 24 hours for initial bacteria amount ranging from 1 to 1000 CFU/mL (Fig. 5a and 5b). For *S. aureus,* regardless of the initial number of bacteria, the presence of nucleases was detected after 24 h of incubation, suggesting that the method of the invention is able to detect as low concentration as 1 CFU/mL (Fig. 5c). At lower incubation time (14 h), we observed that at least 100 CFU/mL of initial bacteria is required to obtain a reasonable value of dispersion degree (D > 5) (Fig. 5c). Note that all the inoculi showed negative results for CFU/mL ranging from 10¹ to 10⁸, suggesting that at 0 h no nucleases are secreted to the media, even at high bacterial concentrations (Fig. 13).

To obtain more quantitative values of the detection limit for nucleases discrimination, we performed a calibration curve using commercial *Micrococcal* Nuclease (Fig. 12), an extracellular nuclease secreted by *S. aureus* (Fig. 9). By taking into the account that LOD = 3Sb / a, where Sb is sample standard deviation of blank and a is mean of blank value, we estimate that the limit of detection of the method of the invention is equal to 12.7 µU/µL (D = 1.8), which is much below the colorimetric, qualitative colorimetric limit (D = 5), as observed after 14 h of incubation of the culture at an initial bacteria amount of 100 CFU/mL.

The detection of *Salmonella* (tested for *S.* Typhimurium) revealed similar trend, but the detection of 1 CFU/mL was not achieved at 24 hours (Fig. 5d) but after 48 hours of incubation (Fig. 10).

Next, we tested *S. aureus* duplex linker (composed by SEQ ID NO: 3 and SEQ ID NO: 8) and *S.* Typhimurium and *S.* Enteritidis duplex linker (composed by SEQ ID NO: 10 and SEQ ID NO: 11) for five food samples (chicken, lettuce, omelette, potato salad, cottage cheese) spiked with these bacteria and in peptone water (BPW) as medium reference. The initial bacteria concentration of 1000 CFU/mL was kept constant in all samples. The supernatants were collected at different incubation times: 0, 4, 8, 14, 18, and 24 hours and tested with the colorimetric assay (Fig. 6). Interestingly, the efficiency of nucleases detection is food-specific. For *S. aureus,* the spiked food samples of cheese, chicken and lettuce showed the presence of nucleases at shorter incubation times, between 4 and 14 h. Omelette revealed the presence of nuclease after 24 h of incubation, while in potato salad no nucleases were detected (Fig. 6a). The detection of *S.* Typhimurium and *S.* Enteritidis in the food samples showed a different pattern: the nucleases was detected already at 4 hours in cheese and potato salad samples, while 24 and 14 hours were required to detect *S.* Typhimurium and *S.* Enteritidis in chicken sample, respectively (Fig. 6b, 6c).

The differences of appearing positive outcome as a function of incubation times for different food sample is due to the availability of nutrients that condition bacterial culture growth and hence secretion of nucleases. Such observation was further confirmed by the fact that nucleases detection in pure medium (BPW) requires 24 hours of incubation to achieve positive results regardless the type of bacteria, suggesting again that the generation of nucleases requires the presence of proper nutrients.

Finally, we tested the method of the invention for the detection of bacterial nucleases in processed food samples suspected to be naturally contaminated by *S. aureus.* Four different food matrices (i.e. two of bread, one of duck and one of pancake) were found as *Staphylococcus* positive in the routine analysis of processed food made at the National Centre for Food Technology and Safety (CNTA). From those, only the duck sample showed a positive result to *S. aureus* with the method of the invention (Fig. 7). This finding was posteriorly confirmed by the standard microbiological tests carried out at the Spanish National Reference Centre of Microbiology Instituto de Salud Carlos III, where the other 3 samples were identified as *Staphylococcus epidermidis*, demonstrating the specificity of the method of the invention. Noteworthy, *Salmonella* was not found contaminating naturally any processed food during the routine controls, thus, this type of experiment cannot be performed.

**Table 3. Non-Salmonella and non-Staphylococcal strains from the IdAB collection used in this work.**

| Code | Characteristic |
|---|---|
| NS1 to NS5 | *Escherichia coli* ATCC 25981 and other 4 strains isolated from food samples and fattening pigs |
| NS6 | *Enterococcus faecalis* isolated from fattening pigs |
| NS7 and NS8 | *Hafnia alvei* isolated from pig environment |
| NS9 | *Moellerella winsconsiensis* isolated from food samples |
| NS10 | *Oceanobacillus oncorhynchi* isolated from food samples |
| NS11 and NS12 | *Pseudomona aeruginosa* isolated from fattening pigs |
| NS13 | *Proteus mirabilis* isolated from fattening pigs |
| NS14 | *Providencia rustigianii* isolated from food samples |
| NS15 | *Proteus vulgaris* isolated from fattening pigs |
| NS16 | *Staphylococcus aureus* 1 isolated from human |
| NS17 and NS18 | *Staphylococcus aureus* isolated from food samples |
| NS19 | *Serratia liquefaciens* isolated from pig environment |
| NS20 | *Streptococcus pyogenes* isolated from food samples |
| NS21 | *Klebsiella pneumoniae* isolated from food samples |

## Claims

1. An *in vitro* method for colorimetric detection of bacterial nucleases in a food sample comprising:
a) providing a hemi-methylated oligonucleotide duplex linker with overhanging ends;
b) mixing the duplex linker of step (a) with a food sample forming a reaction solution;
c) incubating the reaction solution of step (b) for sufficient time to allow cleavage of the duplex linker in case nucleases are present in the reaction solution;
d) providing at least two types of functionalized nanoparticles, wherein the first type of nanoparticle is functionalized with an oligonucleotide probe being complementary to one of the overhanging ends of the duplex linker, and the other type of nanoparticle is functionalized with a different oligonucleotide probe being complementary to the other overhanging end of the duplex linker;
e) adding the nanoparticles obtained in step (d) to the reaction solution from step (c), forming the final solution, and incubate under conditions effective for the hybridization of the duplex linker comprising overhanging ends with the nanoparticle's oligonucleotide probes; and
f) examining the final solution obtained in step (e) wherein if the final solution colour is red due to the dispersion of the nanoparticles, it indicates the presence of the bacterial nucleases in the food sample, while if the solution colour is blue due to the aggregation of the nanoparticles, it indicates the absence of bacterial nucleases in the food sample.

2. The method according to claim 1 wherein the hemi-methylated oligonucleotide duplex linker comprises two strands of RNA or one strand of DNA and one strand of RNA.

3. The method according to any of claims 1 to 2 wherein the oligonucleotide probes content of GC is less than 30%.

4. The method according to any of claims 1 to 3 wherein the oligonucleotide probes comprise a spacer of 18 poly-ethylene-glycol groups located between the thiol group at the 5'end and the nucleotide sequence of the oligonucleotide probe.

5. The method according to any of claims 1 to 4 wherein the nanoparticles are functionalized with 40 to 140 probes in their surface, preferably 50 to 60 probes.

6. The method according to any of claims 1 to 5 wherein the oligonucleotide probes comprise an oligonucleotide sequence selected from the list consisting of: SEQ ID NO 1 and SEQ ID NO: 2.

7. The method according to any of claims 1 to 6 wherein the methylated strand of the hemi-methylated duplex linker comprises an oligonucleotide sequence selected from the list consisting of: SEQ ID NO: 8, SEQ ID NO: 11 and SEQ ID NO: 14; and the non-methylated strand of the duplex linker comprises an oligonucleotide sequence selected from the list consisting of: SEQ ID NO: 3, SEQ ID NO:4, SEQ ID NO:9, SEQ ID NO: 10, SEQ ID NO: 12 and SEQ ID NO:13, preferably wherein the hemi-methylated duplex linker is composed by SEQ ID NO: 8 and SEQ ID NO: 3 or SEQ ID NO: 8 and SEQ ID NO: 4 or SEQ ID NO: 11 and SEQ ID NO: 10 or SEQ ID NO: 14 and SEQ ID NO: 13 or SEQ ID NO: 11 and SEQ ID NO: 9 or SEQ ID NO: 14 and SEQ ID NO: 12.

8. The method according to any of claims 1 to 7 wherein the bacterial nucleases are from *Salmonella spp*., *Staphylococcus spp*., *Listeria spp*., *E. coli*, *Campylobacter spp*., more preferably from *S.* Typhimurium, *S.* Enteritidis, *S. aureus* or any combination of said bacterial nucleases.

9. The method according to any of claims 1 to 8 wherein the nanoparticles are gold or silver nanoparticles, preferably gold nanoparticles, and their morphology is spherical, cube-like, rod-like, bipyramidal, or prism-like, preferably spherical shape.

10. The method according to any of claims 1 to 9 wherein the nanoparticles size is between 15 to 50 nm, preferably between 22 to 30 nm, more preferably at least 22, 23, 24, 25, 26, 27, 28, 29 and 30 nm.

11. The method according to any of claims 1 to 10 wherein step c) is performed at 20 to 45°C during 20 to 110 min, preferably at 30 to 40°C during 15 to 45 min, more preferably at 37°C during 30 min.

12. The method according to any of claims 1 to 11 wherein the concentration of duplex linker in the final solution is 15 to 45 nM, preferably 25 nM to 30 nM.

13. The method according to any of claims 1 to 12 wherein step e) is performed during 20 to 110 min, preferably at 30 to 60 min, more preferably at least 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 min.

14. The method according to any of claims 1 to 13 wherein step (e) is carried out in the presence of a salt at a concentration in final solution of 0.1 to 0.3 M, preferably 0.2 M, and/or step (c) is carried out in the presence of calcium at a concentration of up to 20 mg/L in the reaction mixture, preferably 1 to 5 mg/L, more preferably at least 1, 2, 3, 4, 5 mg/L.

15. A kit for colorimetric detection of bacterial nucleases comprising a least one hemi-methylated oligonucleotide duplex linker with overhanging ends and at least two types of functionalized nanoparticles each type functionalized with a different oligonucleotide probe, wherein the hemi-methylated oligonucleotide duplex linker comprises a methylated strand, preferably selected from a list consisting of: SEQ ID NO: 5, SEQ ID NO: 8, SEQ ID NO: 11 and SEQ ID NO: 14; and a non-methylated strand of the duplex linker comprises an oligonucleotide sequence, preferably selected from the list consisting of: SEQ ID NO: 3, SEQ ID NO:4, SEQ ID NO:9, SEQ ID NO: 10, SEQ ID NO: 12 and SEQ ID NO:13, and the oligonucleotide probe sequence is preferably selected from the list consisting of: SEQ ID NO 1 and SEQ ID NO: 2.

16. The use *in vitro* of a kit according to claim 15 for colorimetric detection of bacterial nucleases according to the method of any of claims 1 to 14.
